# EUROPEAN PATENT APPLICATION

(11) **EP 2 671 544 A2**
(43) Date of publication of application: **11.12.2013**
(21) Application number: 12461543.6
(22) Date of filing: 11.09.2012
(51) Int. Cl.: A61F 5/01

(54) **Method for producing an orthotic brace and orthotic braces produced using this method.**

(30) Priority: 04.06.2012 PL 39939912
(71) Applicant: MTB Poland sp. z o.o., 95-070 Aleksandrow (PL)
(72) Inventor: Herka, Katarzyna, 98-105 Kwiatkowice las (PL); Piotrowski, Andrzej, 91-496 ?ód? (PL)
(74) Representative: Rumpel, Alicja

(57) **Abstract**

The method for producing an orthotic brace is comprised of the following steps:
1. Generation of a 3D image/scan of the injured area.
2. Preparation of a virtual 3D model of the limb.
3. Determination of the use conditions for the orthosis.
4. Preparation of a virtual 3D model of the orthosis and adjustment of the model.
5. 3D printing of the elements of the orthosis frame core (2).
6. Preparation of the composite cover of the frame (1), preferably together with at least one electrostimulation circuit (4).
7. Comparison of the printed elements of the orthosis with the orthosis hinges (5), external elements (6) of the frame (1) and elements of the electrostimulation system.

and the orthotic brace according to the invention, containing a rigid frame (1) with a four-point limb and joint stabilisation system, a polycentric hinge (5), whose composite frame (1) is provided with an electrostimulation circuit (4) with at least two electrodes (7).

## Description

This invention relates to the method for producing an orthotic brace and orthotic braces for active stimulation of the peri-injured area.

In preventive treatment and post-injury rehabilitation there are known and widely used various types of orthotic braces with the main aim of bracing and/or blocking the joint with partial loss of functional ability due to the injury. The loss of functional ability results most often from the damage to the ligaments or muscular attachments which not only causes pain syndromes, but also limits the defensive mechanisms of the joint against uncontrolled movements of the individual parts of the skeleton which are included in the joint.

Orthotic braces in use today are an alternative to plaster casts applied in the past and enable rehabilitation of the injured area almost immediately after the injury occurs. Thus the muscle tissue is not weakened due to the immobility of the limb and any possible secondary injuries caused by hyperextension or excessive mobility of the joint are eliminated as a result of the orthosis bending angle lock.

There is known an orthosis according to the patent description no. US8043243 which is composed of a two-piece frame with arms shaped as two letters L positioned back to front at side view, joined movable via a single-axis hinge provided with a pattern of holes which makes it possible to partially or totally limit the movement range of the knee joint.

A similar possibility is offered by the orthosis disclosed in the American patent no. US6890314, wherein a double-axis hinge was used, and the arms of the orthosis frame were provided with combs (teeth) for regulating the speed of bending of the joint. Similarly as the orthosis referred to above, this orthosis makes it possible to limit or block the movement range of the joint by including into the hinge a lock of size corresponding to the extent of the dysfunction.

These orthoses are mass-produced and their sizes are selected based on the anthropometric atlas developed for a given population. However, the limb with the injured joint is not infrequently temporarily immobilised with a plaster cast immediately after the injury and only after it is removed, it is possible to secure the joint with an orthosis and possibly start rehabilitation. Then it is often determined that proper stabilisation of the orthosis on the limb is more difficult as the muscles are weakened and have reduced mass due to their long-term immobilisation. This is particularly evident in the close proximity of the injured joint which often prevents the application of traditional mass-produced orthoses. Therefore, as soon as the plaster cast is removed, electrostimulation of the muscles is used for their strengthening prior to the proper rehabilitation.

For this reason, in the case of permanently immobilised limbs and significant difference between the size of the patient's limb and the standard orthosis sizes offered by the manufacturer, especially in the event of post-surgical ACL and PCL reconstructions and injuries associated with MCL and LCL elongation or rupture, customised orthoses are made based on the taken limb measurements. This orthosis is usually carried out as a traditional splint-and-strap orthosis, in which each arm is made and bent according to the taken measurements and several fittings of the orthosis. It is the necessity of multiple measurement-taking and fitting operations that generates the high costs (mostly labour costs) of such orthoses as any deviation from the planned size and geometry of the orthosis may cause discomfort or destabilisation of the joint. However, fittings are each time associated with significant losses of material and the need to make several orthosis frames, where only one is used.

Additionally, in the case of some patients it is necessary to apply biochemically neutral materials on account of skin diseases or the type of the physical therapy. Also the weight of the orthosis is a very important factor, and therefore frames are made of such materials as surgical steel, aluminium and also composite materials.

This orthosis is known for example from the patent description no. US4517968, wherein a design for ankle, wrist, elbow or knee orthosis was disclosed, and the fragments of the orthosis frame on the same side of the limb are fastened with fasteners in the form of Velcro straps and the entire orthosis is stabilised on the limb with bandage. Such design of the orthosis enables to the maximum extent proper customisation of the orthosis shape to the shape and size of the limb. The method of their installation on the lower limb causes that the orthoses of this design resemble splints and orthopaedic devices for permanent immobilisation of limbs and similarly limit to a significant extent the movement range of the joint.

Therefore, it was appropriate to develop a method for producing an orthosis which would enable reducing the costs and losses of material and at the same time provide functionality and full customisation of the end product to the limb so as to be able to start rehabilitation at the stage of its full or partial immobilisation.

The method for producing an orthotic brace and orthotic braces produced using the method according to the invention offers such possibility.

The method for producing an orthotic brace according to the invention is comprised of the following steps:
1. Generation of a 3D image/scan of the injured area.
2. Preparation of a virtual 3D model of the limb.
3. Determination of the use conditions for the orthosis.
4. Preparation of a virtual 3D model of the orthosis and adjustment of the model.
5. 3D printing of the elements of the orthosis frame core.
6. Preparation of the composite cover of the frame, preferably together with at least one electrostimulation circuit.
7. Comparison of the printed elements of the orthosis with the orthosis hinges, external elements of the frame and elements of the electrostimulation system.

The 3D image / scan of the injured area is generated using any known method of image diagnostics, preferably with at least an X-ray apparatus or CAT scanner, preferably coupled with CAD or similar software, preferably with at least an X-ray apparatus and CAT scanner or preferably with a laser scanner in another example of embodiment. During the generation of the 3D image / scan of the injured area at least the following measurements are taken: circumference above and below the joint, circumference of the joint, length of the orthosis.

The image of the limb and the injured area is then recorded in digital format as a virtual three-dimensional model. Preferably, at the time of generation of the virtual three-dimensional model, the use conditions for the orthosis are determined, and in particular: weight of the user, type of injury and the resulting limitations of the movement range of the limb and the joint - specified at least as the minimum and maximum flexion and extension angles, and also the intensity of use as at least the determination of the rehabilitation method.

Based on the parameters from image examinations as well as the taken measurements and the determined use conditions for the orthosis a virtual three-dimensional model of the orthosis is generated which is overlaid on the earlier generated 3D model of the injured area. This enables adjusting the model of the orthosis without seeing the patient. After the three-dimensional model of the orthosis is accepted, the elements of the orthosis core are printed with a 3D printer. The 3D print is made of acrylonitrile-butadiene-styrene (ABS) or polyamide powders.

The external cover of the frame is made as a composite of natural fibres with the use of resin preferably selected from among polymer resins, e.g. epoxy, polyester, polyurethane or acrylic resins. Natural fibres are preferably flax, cocoa or bamboo fibres. During the production of the external cover of the frame at least one electric circuit with at least two terminals of electrodes for muscle stimulation and preferably a connector for controlling the electrostimulation/electric circuit is embedded in its structure.

In another example of embodiment the external cover of the frame is also preferable made as a composite of natural fibres with the use of resin preferably selected from among polymer resins, e.g. epoxy, polyester, polyurethane or acrylic resins. The core of the frame is braided with natural fibres using a plaiting machine. During the production of the external cover of the frame at least one electric circuit with at least two terminals of electrodes for muscle stimulation and preferably a connector for controlling the electrostimulation/electric circuit is embedded in its structure.

Then the core of the frame with the composite cover, and the hinges are put together. At this stage, in the structure of the frame, at least one of the circuits provided in the frame is connected to the electrode terminals and preferably, in the event that a connector for controlling the electrostimulation is used, at least one controller is connected to the electric circuit. A portion of the composite cover of the frame in contact with the limb is preferably provided additionally with a soft subframe to enable adjusting the circumference of the orthosis to the limb.

The orthotic brace according to the invention contains a rigid frame with a four-point limb and joint stabilisation system, a polycentric and at least double-axis hinge, and also at least one circuit for muscle electrostimulation, provided with at least two electrodes. The rigid frame contains at least two 3D prints which constitute the core of the frame as well as the composite cover of the frame elements, and the composite cover of the frame contains at least one electric circuit, provided with at least two electrodes and embedded in its surface. The electric circuit is preferably provided with a connector for connecting the controller of muscle electrostimulation.

The frame of the orthosis according to the invention is made as a composite with a core printed in 3D technology, and the composite cover of the orthosis frame is composed of natural fibres, preferably flax, bamboo or cocoa fibres and resin. The polycentric hinge put together with the frame is provided with at least a double-sided removable lock to limit the angular movement range of the joint. The polycentric hinge put together with the frame is preferably provided with at least a double-sided lock to limit the angular movement range of the joint.

The electric circuit provided in the orthosis frame enables acting on the muscle tissue with electrical impulses or, if at least two electric circuits are used in another example of embodiment - with interferential currents.

Electrical impulses allow stimulating the muscles and their training without straining the ligaments and the joint, while the interferential currents penetrate deep into the tissue without causing any drop in the voltage when passing through the skin and alleviate pain and also improve the vascularity and nerve conduction in the motor nerves as well as the sensory threshold of the sensory nerves.

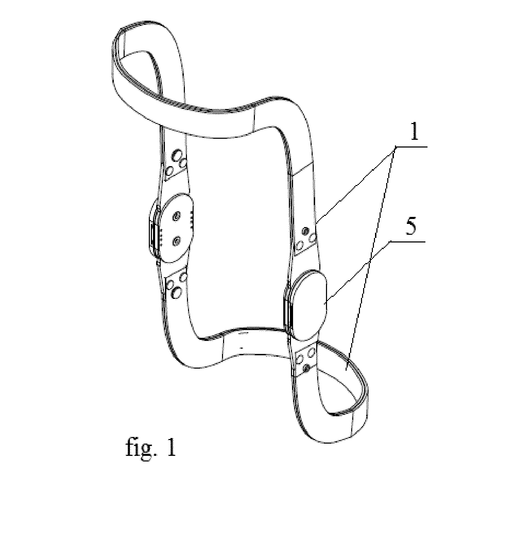

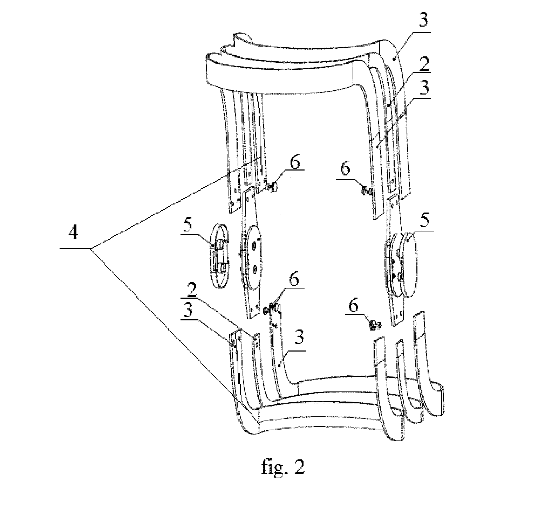

The orthotic brace according to the invention is presented in the figures, where Fig. 1 shows the orthosis and Fig. 2 shows the design of the orthosis according to the invention and the first example of embodiment.

### Example I

The method for producing an orthotic brace according to the invention is comprised of the following steps:
1. Generation of a 3D image/scan of the injured area.
2. Preparation of a virtual 3D model of the limb.
3. Determination of the use conditions for the orthosis.
4. Preparation of a virtual 3D model of the orthosis and adjustment of the model.
5. 3D printing of the elements of the orthosis frame core.
6. Preparation of the composite cover 3 of the frame 1, preferably together with at least one electrostimulation circuit 4.
7. Comparison of the printed elements of the orthosis with the hinges 5 of the orthosis.

The 3D image / scan of the injured area is generated using image diagnostic methods, and during the generation of the 3D image / scan of the injured area the geometric parameters of the orthosis are determined.

The image of the limb and the injured area is then recorded in digital format as a virtual three-dimensional model. At the time of generation of the virtual three-dimensional model, the use conditions for the orthosis are determined, and in particular: weight of the user, type of injury and the resulting limitations of the movement range of the limb and the joint, and also the intensity of use of the orthosis.

Based on the parameters from image examinations as well as the taken measurements and the determined use conditions for the orthosis a virtual three-dimensional model of the orthosis is generated which is overlaid on the earlier generated 3D model of the injured area. This enables adjusting the model of the orthosis without seeing the patient. After the three-dimensional model of the orthosis is accepted, the elements of the orthosis core are printed with a 3D printer.

Then the composite cover 3 of the frame 1 core 2 is made as a composite of natural fibres with the use of resin. During the production of the composite cover 3 of the frame 1 core 2 an electric circuit 4 with terminals of electrodes for muscle stimulation 7 and a connector 8 for controlling the electrostimulation circuit is embedded in its structure.

Then the core 2 of the frame 1 with the composite cover 3 and the hinges 5 are put together. At this stage, in the structure of the frame 1, the circuit 4 provided in the frame is connected to the electrode 7 terminals and a controller is connected to the electric circuit connector. A portion of the composite cover 3 of the frame 1 in contact with the limb is additionally provided with a soft subframe to enable adjusting the circumference of the orthosis to the limb.

The orthotic brace according to the invention contains a rigid frame 1 with a four-point limb and joint stabilisation system, polycentric and double-axis hinges 5, and also at least one muscle electrostimulation circuit 4 provided with electrodes 7. The rigid frame 1 contains 3D prints which constitute the core 2 of the frame as well as the composite cover 3 of the frame 1 core 2. The composite cover 3 of the frame 1 has an electric circuit 4 provided with electrodes and embedded in its surface. The electric circuit 4 is provided with a connector 8 for connecting the muscle electrostimulation controller.

The frame 1 of the orthosis according to the invention is made as a composite with a core 2 printed in 3D technology, and the composite cover 3 of the orthosis frame 1 is composed of natural fibres and resin. The polycentric hinges 5 put together with the frame 1 is provided with a lock to limit the angular movement range of the joint.

The electric circuit 4 provided in the frame 1 of the orthosis enables acting on the muscle tissue with electrical impulses.

Electrical impulses allow stimulating the muscles and their training without straining the ligaments and the joint, while the interferential currents penetrate deep into the tissue without causing any drop in the voltage when passing through the skin and alleviate pain and also improve the vascularity and nerve conduction in the motor nerves as well as the sensory threshold of the sensory nerves.

### Example II

The method for producing an orthotic brace according to the invention is comprised of the following steps:
1. Generation of a 3D image/scan of the injured area.
2. Preparation of a virtual 3D model of the limb.
3. Determination of the use conditions for the orthosis.
4. Preparation of a virtual 3D model of the orthosis and adjustment of the model.
5. 3D printing of the elements of the orthosis frame 1 core 2.
6. Preparation of the composite cover 3 for the frame 1, preferably together with at least one electrostimulation circuit.
7. Comparison of the printed elements of the orthosis with the hinges 5 of the orthosis.

The 3D image / scan of the injured area is generated using image diagnostic methods, and during the generation of the 3D image / scan of the injured area the geometric parameters of the orthosis are determined.

The image of the limb and the injured area is then recorded in digital format as a virtual three-dimensional model. At the time of generation of the virtual three-dimensional model, the use conditions for the orthosis are determined, and in particular: weight of the user, type of injury and the resulting limitations of the movement range of the limb and the joint, and also the intensity of use of the orthosis.

Based on the parameters from image examinations as well as the taken measurements and the determined use conditions for the orthosis a virtual three-dimensional model of the orthosis is generated which is overlaid on the earlier generated 3D model of the injured area. This enables adjusting the model of the orthosis without seeing the patient. After the three-dimensional model of the orthosis is accepted, the elements of the orthosis core are printed with a 3D printer.

Then the composite cover 3 of the frame 1 core 2 is made as a composite of natural fibres with the use of resin. The core 2 of the frame 1 is braided with natural fibres using a plaiting machine, and during the production of the external elements 6 of the frame 1 an electric circuit 4 with terminals of electrodes for muscle stimulation 7 and a connector 8 for controlling the electrostimulation circuit is embedded in its structure.

Then the core 2 of the frame 1 with the composite cover3 and the hinges 5 are put together. At this stage, in the structure of the frame 1, the circuit 4 provided in the frame is connected with electrode 7 terminals and a controller is connected to the electric circuit connector. A portion of the composite cover 3 of the frame 1 in contact with the limb is additionally provided with a soft subframe to enable adjusting the circumference of the orthosis to the limb.

The orthotic brace according to the invention contains a rigid frame 1 with a four-point limb and joint stabilisation system, polycentric and double-axis hinges 5, and also at least one muscle electrostimulation circuit 4 provided with electrodes 7. The rigid frame 1 contains 3D prints which constitute the core 2 of the frame as well as the composite cover 3 of the frame 1 core 2. The composite cover 3 of the frame 1 core 2 has an electric circuit 1 provided with electrodes and embedded in its surface. The electric circuit 4 is provided with a connector 8 for connecting the muscle electrostimulation controller.

The frame 1 of the orthosis according to the invention is made as a composite with the core 2 printed in 3D technology, and the composite cover 9 of the orthosis frame 1 is composed of natural fibres and resin. The polycentric hinges 5 put together with the frame 1 is provided with a lock to limit the angular movement range of the joint.

The electric circuit 4 provided in the frame 1 of the orthosis enables acting on the muscle tissue with electrical impulses.

Electrical impulses allow stimulating the muscles and their training without straining the ligaments and the joint, while the interferential currents penetrate deep into the tissue without causing any drop in the voltage when passing through the skin and alleviate pain and also improve the vascularity and nerve conduction in the motor nerves as well as the sensory threshold of the sensory nerves.

## Claims

1. The method for producing an orthotic brace **characterised in that** it is comprised of the following steps:
1. Generation of a 3D image/scan of the injured area.
2. Preparation of a virtual 3D model of the limb.
3. Determination of the use conditions for the orthosis.
4. Preparation of a virtual 3D model of the orthosis and adjustment of the model.
5. 3D printing of the elements of the orthosis frame (1) core (2).
6. Preparation of the composite cover (3) of the frame (1), preferably together with at least one electrostimulation circuit (4).
7. Comparison of the printed elements of the orthosis with the orthosis hinges (5), cover (3) of the frame (1) core (2) and the elements of the electrostimulation system.

2. A method according to claim 1 **characterised in that** the 3D image / scan of the injured area is generated using any known method of image diagnostic method using devices selected from among an X-ray apparatus or CAT scanner and laser scanner, and when the 3D image / scan of the injured area is generated, at least the following parameters of the injured area are determined: circumference above and below the joint, circumference of the joint length of the orthosis and at the same time a virtual three-dimensional model of the limb / injured area is generated and the use conditions for the orthosis are determined.

3. A method according to claims 1 or 2 **characterised in that** a virtual three-dimensional model of the orthosis is overlaid on the image / scan of the injured area and the former is adjusted based on the parameters from image examinations as well as the taken measurements and the determined use conditions for the orthosis.

4. A method according to any of the claims referred to above **characterised in that** after the three-dimensional model of the orthosis is accepted, elements of the core (2) of the frame (1) of the orthosis are printed using a 3D printer, on which the composite cover (3) of the frame (1) core (2) is provided based on the results of image examinations.

5. A method according to claim 1 or 4 **characterised in that** after the composite cover (3) of the frame (1) core (2) is made of natural fibres selected from among flax, bamboo or cocoa fibres, and resin selected from among polymer resins, in particular epoxy, polyester, polyurethane or acrylic resins, is used as an adhesive.

6. A method according to claim 1 or 4 or 5 **characterised in that** during the production of the composite cover (3) of the frame (1) core (2) at least one electric circuit (4) with at least two terminals of electrodes (6) for muscle stimulation is embedded in its structure.

7. A method according to claim 1 or 4 or 5 **characterised in that** during the production of the external elements (6) of the frame (1) at least one electric circuit (4) with at least two terminals of electrodes (7) for muscle stimulation and a connector (8) for controlling the electrostimulation circuit (4) is embedded in their structure.

8. A method according to claim 5 or 6 or 7 **characterised in that** the external cover (3) of the external elements (6) of the frame (1) is made as a composite with its non-woven fabric structure braided with natural fibres placed on the core printed in 3D technology using a plaiting machine.

9. An orthotic brace according containing a rigid frame (1) with four-point limb and joint stabilisation system, a polycentric hinge (5), **characterised in that** it contains at least one circuit (4) for muscle electrostimulation, provided with at least two electrodes (7), and the rigid frame (1) of the orthosis contains at least two 3D prints which constitute the core (2) of the frame (1) and a composite cover (3) of the frame (1) elements, and the composite cover (3) of the frame has at least one electric circuit (4) with at least two electrodes (7), embedded in its surface.

10. An orthotic brace according to claim 9 **characterised in that** the electric circuit (4) is provided with a connector (8) for connecting a muscle electrostimulation controller.

11. An orthotic brace according to claim 9 or 10 **characterised in that** the frame (1) of the orthosis according to the invention is made as a composite with a core (2) printed in 3D technology, and the composite cover (3) of the orthosis frame (1) is composed of natural fibres, and the frame (1) with the cover (3) is put together with a polycentric hinge (5) provided with at least a double-sided removable lock to limit the angular movement range of the joint.
